(19) **Europäisches Patentamt**
**European Patent Office**
**Office européen des brevets**

(11) **EP 2 042 182 B1**

(12) **EUROPEAN PATENT SPECIFICATION**

(45) Date of publication and mention
of the grant of the patent:
**22.05.2013 Bulletin 2013/21**

(51) Int Cl.:
*A61K 33/36* [(2006.01)]  *A61P 35/00* [(2006.01)]

(21) Application number: **08168243.7**

(22) Date of filing: **10.04.2002**

(54) **Pharmaceutical composition comprising arsenite for the treatment of malignancy**

Pharmazeutische Zusammensetzung mit Arsenit zur Behandlung von Malignomen

Composition pharmaceutique comprenant de l'arsénite pour le traitement de tumeurs

(84) Designated Contracting States:
**AT BE CH CY DE DK ES FI FR GB GR IE IT LI LU MC NL PT SE TR**

(43) Date of publication of application:
**01.04.2009 Bulletin 2009/14**

(62) Document number(s) of the earlier application(s) in accordance with Art. 76 EPC:
**02722968.1 / 1 496 918**

(73) Proprietor: **Komipharm International Co., Ltd.**
**Jungwang-Dong**
**Shihung City**
**Kyonggi do (KR)**

(72) Inventor: **Rademaker, Bernardus**
**Onstwedde (NL)**

(74) Representative: **Sharples, Andrew John et al**
**EIP**
**Fairfax House**
**15 Fulwood Place**
**London WC1V 6HU (GB)**

(56) References cited:
**EP-A- 0 804 928**   **WO-A-99/18798**

- **KIM H-S ET AL: "Intracellular Glutathione Level Modulates the Induction of Apoptosis by DELTA-Prostaglandin J2" PROSTAGLANDINS, BUTTERWORTH, STONEHAM, MA, US, vol. 51, no. 6, 1 June 1996 (1996-06-01), pages 413-425, XP004032382 ISSN: 0090-6980**
- **WAXMAN, S., ANDERSON, K.C.: "History of the Development of Arsenic Derivatives in Cancer Therapy" THE ONCOLOGIST, vol. 6, no. 2, 2001, pages 3-10, XP001087583**

Remarks:
The file contains technical information submitted after the application was filed and not included in this specification

EP 2 042 182 B1

**Description**

The present invention relates to a pharmaceutical composition containing arsenic for the treatment of a malignancy.

Pharmaceutical compositions containing arsenic are known for cancer therapy. For example, the review by Waxman S. et al (The Oncologist 6 (suppl. 2), pp. 3-10 (2001) ) describes arsenic disulfide, arsenic trisulfide and arsenic trioxide the use of said composition comprising a salt of meta-arsenite ($AsO_2^-$) and a pharmaceutically acceptable auxiliary.

Due to their inherent toxicity, and the advent of good alternatives, interest in arsenic compounds has remained low.

The object of the present invention is to provide a pharmaceutical composition suitable for use in cancer therapy, which allows for the treatment of solid tumours. The pharmaceutical composition may be used for treatment of such solid tumours for which currently no treatment exists, or as an alternative or supplementary treatment. for such solid tumours.

To this end, the pharmaceutical composition according to the present invention is characterized in that it is a pharmaceutical composition for the treatment of a solid malignancy chosen from the group consisting of colon tumour, gastric tumour, mammary tumour, ovarian tumour, prostate tumour, and renal tumour, said composition comprising a salt of meta-arsenite ($AsO_2^-$) and a pharmaceutically acceptable auxiliary.

It has been found that the specified types of tumour are surprisingly sensitive to the meta-arsenite salt. In the salt the counter-ion of meta-aresenite may be any pharmaceutically acceptable counter-ion.

In the article by Waxman, mention is made of an article by Tarnowski G. S. et al (Cancer Research, 26(2), pp. 181-206 (1966) ) where 8 tumour types were investigated with 14 different anti-tumour chemicals. Potassium arsenite affected only the growth of Ehrlich ascites tumour. Regarding this tumour type it is remarked that it is more sensitive in the ascites than in the sold form. This emphasizes the surprising finding of the present invention.

According to a preferred embodiment, the salt is an alkaline or earth alkaline metal salt.

According to a more preferred embodiment, the alkaline metal salt is a potassium or sodium salt.

Such salts are readily soluble and are readily available for exerting their anti-tumour effect.

The invention also relates to the use of a salt of meta-arsenite ($AsO_2^-$) for the manufacture of a pharmaceutical composition for the treatment of a solid malignancy chosen from the group consisting of colon tumour, gastric tumour, mammary tumour, ovarian tumour, prostate tumour, and renal tumour.

It was found that solid malignancy belonging to the group consisting of colon tumour, mammary tumour, prostate tumour, and renal tumour were particularly sensitive.

Finally, the present invention relates to a method of treating a human individual suffering from a solid malignancy chosen from the group consisting of colon tumour, gastric tumour, mammary tumour, ovarian tumour, prostate tumour, and renal tumour, with a pharmaceutically effective dose of a salt of meta-arsenite ($AsO_2^-$).

The present invention will now be elucidated with reference to the following non-limiting example.

EXAMPLE

Various human tumor cells were grown at 37 C in a humidified atmosphere (95% air, 5% CO2) in monolayer cultures in RPMI 1640 medium with phenol red (Life Technologies, Karlsruhe, Germany) supplemented with 10% fetal calf serum.

Cells were trypsinized and maintained weekly.

Cytotoxicity assay

A modified propidium iodide assay (based on W. A. Dengler et. al, Anti-Cancer Drugs, 6, pp. 522-532 (1995) ) was used to examine the antiproliferative activity of the study compounds. Briefly, cells will be harvested from exponential phase cultures growing in RPMI 1640 medium supplemented with 10% fetal calf serum by trypsination, counted and plated in 96 well flat-bottomed microtiter plates (140 $\mu$l cell suspension, 8 x $10^4$ cells/ml). After a 24h recovery, to allow cells to resume exponential growth, 10 $\mu$l culture medium (6 control wells per plate) or culture medium containing the test drug were added to the wells. Each drug concentration was plated in triplicate. After 4 days of incubation culture medium was replaced by an aqueous propidium iodide solution (6 $\mu$g/ml). Microtiter plates were kept at -18°C for 24 h, resulting in a total cell kill. After thawing of the plates, fluorescence was measured using a Millipore Cytofluor 2350-microplate reader (excitation 530 nm, emission 620 nm) in order to quantify the total cell number. The assay included untreated and positive controls (5-FU and vindesine).

Growth inhibition is expressed as Treated/Control x 100 (or T/C%). $IC_{50}$ and $IC_{70}$ values were determined by plotting compound concentration versus cell number. Mean $IC_{50}$ and $IC_{70}$ values were calculated according to the formula:

$$\text{Mean IC}_{50,\,70} = \frac{\sum\limits_{x=1}^{n} \log (\text{IC}_{50,\,70})_x}{10^n}$$

With x = specific tumor cell line and n = total number of cell lines studied. If $IC_{50}$ or $IC_{70}$ could not be determined within the examined dose range, the lowest or highest concentration studied was used for the calculation.

Assays were considered valid only if the positive control (5-FU) induced a tumor growth inhibition of T/C < 30% and if vehicle treated control cells had a fluorescence intensity > 500 units.

Results

The results have been summarized in Table I, which shows that in particular tumour cell lines of the type gastric tumour, ovarian tumour, and in particular prostate tumour, mammary tumour, renal and colon tumour were sensitive to the meta-arsenite compound. In comparison, promyelocytic leukaemia, which is known to respond to arsenic trioxide, showed an $IC_{70}$ value of 6.82 $\mu$g/ml. Hence, the tumour cells to which the present invention relates are about 2 to 20 times more sensitive to the meta-arsenite compound according to the present invention.

TABLE I

| Tumour | Cell line | $IC_{70}$ ($\mu$g/ml) |
|---|---|---|
| Colon | DLD1 | 0.48 |
| Gastric | GXF251L | 3.08 |
| Mammary | MXAF401NL | 0.32 |
| Ovarian | OVCAR3 | 3.02 |
| Prostate | PC3 | 0.85 |
| Renal | RXF486L | 0.63 |

## Claims

1.    A pharmaceutical composition comprising arsenic for use for the treatment of a malignancy, wherein said arsenic is sodium meta-arsenite, and said malignancy is a solid tumour.

2.    A pharmaceutical composition according to claim 1, wherein said composition is a supplementary treatment.

## Patentansprüche

1.    Eine pharmazeutische Zusammensetzung umfassend Arsen zur Verwendung für die Behandlung einer Bösartigkeit, wobei erwähntes Arsen ein Natriummetaarsenit und die erwähnte Bösartigkeit eine feste Wucherung ist.

2.    Eine pharmazeutische Zusammensetzung nach Anspruch 1, wobei besagte Zusammensetzung eine ergänzende Behandlung ist.

## Revendications

1.    Composition pharmaceutique comprenant de l'arsenic pour être utilisée dans le traitement d'une malignité, dans laquelle ledit arsenic est du meta-arsenite de sodium et dans laquelle ladite malignité est une tumeur solide.

2.    Composition pharmaceutique selon la revendication 1 dans laquelle ladite composition est un traitement complémentaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WAXMAN S. et al.** *The Oncologist,* 2001, vol. 6 (2), 3-10
- **TARNOWSKI G. S. et al.** *Cancer Research,* 1966, vol. 26 (2), 181-206
- **W. A. DENGLER.** *Anti-Cancer Drugs,* 1995, vol. 6, 522-532